# EUROPEAN PATENT SPECIFICATION

(11) **EP 2 004 235 B1**
(45) Date of publication and mention of the grant of the patent: **24.03.2010**
(21) Application number: 07733952.1
(22) Date of filing: 13.03.2007
(51) Int. Cl.: A61K 9/00, A61K 8/00, A61K 47/02, A61P 1/00, A61K 31/12, A61K 31/70

(54) **COMPOSITION FOR COSMETIC OR PHARMACEUTICAL-DERMATOLOGICAL USE**
ZUSAMMENSETZUNG ZUR KOSMETISCHEN ODER PHARMAZEUTISCH-DERMATOLOGISCHEN VERWENDUNG
COMPOSITION POUR APPLICATION COSMÉTIQUE OU PHARMACO-DERMATOLOGIQUE

(30) Priority: 13.03.2006 IT PD20060082
(43) Date of publication of application: 24.12.2008
(73) Proprietor: Pharmaland S.A., Borgo Maggiore (SM)
(72) Inventor: MARTELLI, Laura, I-35126 Padova (IT); MARTELLI, Mario, I-35126 Padova (IT)
(74) Representative: Cattaneo, Elisabetta
(86) International application number: PCT/IB2007/000598
(87) International publication number: WO 2007/105071

(56) References cited:
- WO-A-2005/058314
- GB-A- 2 092 001
- US-A1- 2006 039 887
- DATABASE WPI Week 200511 Derwent Publications Ltd., London, GB; AN 2005-097127 XP002463738 & KR 2004 081 248 A (ECO-TECH) 21 September 2004 (2004-09-21)
- WATANABE S ET AL: "SUPPRESSIVE EFFECT OF CURCUMIN ON TRICHLOROETHYLENE-INDUCED OXIDATIVE STRESS" JOURNAL OF NUTRITIONAL SCIENCE AND VITAMINOLOGY, UNIVERSITY OF TOKYO PRESS, TOKYO, JP, vol. 46, no. 5, October 2000 (2000-10), pages 230-234, XP009042819 ISSN: 0301-4800

## Description

### Field of the invention

The present invention relates to compositions for cosmetic or pharmaceutical-dermatological use, suitable for maintaining skin cells at, or helping to restore skin cells to, their basal physiological state, enabling them to effect a regeneration of the skin.

### State of the art

For humans, it is a reality that the skin's well-being involves both a pathological aspect and one of aesthetics and cosmetics. In this respect, there is now an established tendency in contemporary society to not only counter pathologies of the skin but also, for aesthetic reasons, to resist entirely natural processes such as formation of wrinkles.

These phenomena in any case have in common a general impoverishment of dermal and epidermal functional characteristics. Damage to the skin, though, is nearly always the result of various concomitant causes, some of which are due to external agents, such as ultraviolet radiation or environmental contaminants, while others are ascribable to factors within the body and are mostly linked to natural ageing processes, a weakening of the dermal-epidermal interface or the exacerbation of pathologies which manifest themselves at skin level.

Very often these factors act simultaneously on the skin and can lead to various degrees of cellular distress in the epidermis, which, in time, exhibits changes such as loss of elasticity (elastosis) and wrinkles.

From this the need arises to maintain skin cells at, or contribute to restoring skin cells to, their basal physiological state, enabling them to effect a regeneration of the skin.

There are countless known compositions whose aim is precisely to resist the appearance of the aforesaid ageing phenomena, though their level of effectiveness is not currently such that the aforementioned need is satisfied.

### Summary of the invention

The Applicants have now found that the aforesaid results are obtained with the composition of the present invention comprising:
- curcumin,
- fructose 1-6 diphosphate,
- a metal or basic oxide, or a biologically acceptable salt thereof, or its labile coordination compound able to form, with at least one of said compounds: complexes, derivatives or associations which enhance their activity,

in combination with excipients and/or diluents normally employed for cosmetic or pharmaceutical-dermatological use.

Indeed, said compositions are able to restore skin cells to their basal physiological state, enabling them to effect a regeneration of the dermis and epidermis as demonstrated by experiments undertaken at both the cellular and clinical levels.

The present invention therefore provides formulations for cosmetic use comprising the aforesaid compositions, in particular for preventing wrinkle formation and able to prevent elastosis.

The present invention also provides compositions in the form of a medicament for dermatological use, particularly for treating skin pathologies where it is essential to block the inflammatory process, by modulating the calcium and free radical channels caused by oxidative processes, in order to achieve skin regeneration.

### Detailed description of the invention

Curcumin, characterized by the following formula:

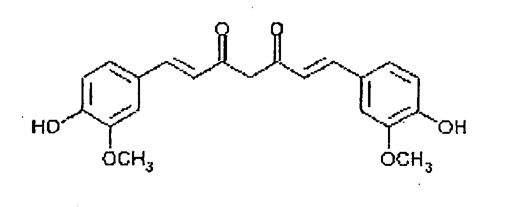

is a natural extract of *Curcuma longa* or *Curcuma xanthorrhiza,* known for its generic antibacterial, antifungal and antiparasitic activity (Ars Pharmaceutica 2000, 41(3), 307-321).

The pharmacological characteristics (Planta medica, 1991, 57, 1) of both the pure product and all analogues (curcuminoids) derived from the extraction process (J. Cellular Biochem. 1996, 265, 72) as well as its safety of use in medicine, have been known for some time.

Curcumin and curcuminoids also behave as muscle relaxants (Life Science 2005, 76, 3089), inhibit the activation of nuclear factor NFkB where the paths causing and sustaining inflammation converge (J. Biol. Chem. 1995, 270, 24995), and are able to inactivate ROS (Reactive Oxygen Species) particularly superoxide ions (Ann. Chim. 2002, 92, 281).

Curcumin is preferably present in the composition of the invention at concentrations between 0.0005% and 10% on the total composition weight.

The phosphosaccharide used in the composition of the present invention is preferably chosen from the group consisting of mannose, glucose, galactose and fructose phosphates. Fructose 1-6 diphosphate (abbreviated to FDP hereinafter) is particularly preferred.

The stated phosphosaccharides, and in particular FDP, are metabolites of glycolysis able to provide easily available energy for cell biochemistry. FDP also possesses prostaglandin E2 (PGE2) and cyclooxygenase inhibitory activity, and is able to preserve the antioxidative capacity of keratinocytes irradiated with ultraviolet B radiation (British J. Pharmacol. 2002, 137, 497). Furthermore, in the presence of sodium and magnesium ions, FDP is accredited with protecting neurones from ischemic attack (Yao Xue Bao. 2003, 38, 325) and cells from various noxae, and facilitating metabolic recovery in ischemic tissue even in conditions of hypoxia (Am. J. Physiol. 1994, 267, H 2325). In this respect, FDP is used mainly for ischemic myocarditis where it interacts with the plasma membrane and stimulates enrichment of the energy-rich intracellular phosphate pool, including 2-3 diphosphogluconate (Esafosfina - information from Biomedica Foscama).

Curcumin and phosphosaccharides chosen from the group specified above have surprisingly shown a good synergistic effect when used in combination, enabling skin cells to recover as much as possible their basal level of efficiency.

Preferably phosphosaccharide is used in concentrations between 0.001% and 25% by weight on the total composition weight.

In accordance with a preferred embodiment, the compositions of the present invention contain the salts or biologically acceptable oxides of a metal able to form, with at least one of the aforesaid essential compounds, coordination compounds or associations which enhance their activity.

The metals contained in the salts or oxides possibly present are chosen from calcium, magnesium, copper, bismuth, zinc, aluminium, manganese, antimony, tin, gold, silver, chromium, cobalt, vanadium or titanium.

Preferably the compositions of the present invention contain oxides or salts of the aforesaid metals able to completely or partially complex curcumin.

If the aforesaid salts or oxides are present in quantities such that the relative metals completely complex curcumin, the compositions of the present invention contain only complexed curcumin.

Instead, if the aforesaid salts or oxides are present in quantities such as to partially complex curcumin, the compositions of the present invention comprise an association of curcumin and complexed curcumin.

This latter type of composition gave the best results in terms of skin cell regeneration following oxidative stress.

Preferred are zinc salts and in particular zinc acetate and zinc oxide.

Formation of the zinc-curcumin complex can be achieved by dissolving the zinc salt in a hydroalcoholic solution to which curcumin is added in a 1:1 molar ratio.

The zinc ion coordinates to the keto-enolic group of curcumin. The same result is obtained by directly adding curcumin to the composition of the invention which contains zinc salts or zinc oxide, as demonstrated by the bathochrome effect, seen in the colour of the compositions after addition of said compound.

Coordination of curcumin with the metal increases its lipophilicity, therefore raising its capacity to act at the cellular level.

In accordance with a further aspect of the present invention, further so-called functional compounds can be present in the composition, useful for sustaining and directing cellular activity when the recovery of basal-physiological conditions indicates full cellular activity has been restored.

In this case, the composition of the present invention will be enriched with one or more compounds chosen from the group consisting of fruit acids, (α-hydroxy acids), glucosaminoglycans, urea, urea in protein mixtures, flavones, flavonoids, terpenes, diterpenes, vaseline, saturated and unsaturated fatty acids, lipids, phospholipids, coumarin and derivates, proteins, protides, amino acids, vitamins, in particular D and H group, ceramides, sphingosines, boswellic acids and derivatives, in particular those characterized by acetyl and formyl groups, starch and its derivatives, as well as monosaccharides.

The composition of the present invention can also advantageously contain one or more compounds, in pharmaceutically compatible doses, chosen from the group consisting of pyrithione and its complex salts (in particular salts of the aforelisted metals), fumaric acid derivatives, Mahonia extracts, anthraquinone derivatives, retinoic acid derivatives, vitamin D derivatives and lactoferrins. In this case the composition will prove to be an excellent coadjuvant in the therapeutic treatment of psoriasis and other skin diseases, avoiding or in any case limiting the use of steroidal substances.

Some examples of formulations in accordance with the invention are given below, as well as clinical trials and in vitro cell trials which demonstrate the effectiveness of the compositions of the present invention. In the examples, 100 grams of a prepared product produced in accordance with the present invention comprise a lipophilic-based excipient in which the stated components are dispersed.

### EXAMPLE 1

- Curcumin 0.002 g
- FDP 4.0 g
- Zinc acetate 0.01 g

### EXAMPLE 2

- Curcumin 0.002 g
- FDP 4.0 g

### EXAMPLE 3

- Curcumin 0.002 g
- FDP 4.0 g
- Zinc acetate 0.005 g

### EXAMPLE 4

- Curcumin 0.002 g
- FDP 4.0 g
- Zinc oxide 0.01 g

### TEST 1

The prepared product of example 1 was used in a test conducted on a sample of twenty female volunteer patients aged between 20 and 25 years with unblemished skin, after obtaining their informed consent regarding the test method.

The prepared product was spread onto one arm while a placebo prepared product, formed solely of a lipophilic-based excipient, was applied to the opposite arm. The entire experiment was conducted "double blind", the codes relating to the placebo and prepared product being opened only at the end of the study.

Clinical assessments were undertaken at the start and at the end of the trial. The parameters relating to the assessment, which was conducted by non-invasive biophysical measurements, were: dryness, irritation and scaling of the skin. In particular, skin hydration was assessed by measuring the electrical capacitance of the skin using the corneometer CM 820 PC (Courage and Khazaka Electronic GmbH, Cologne, Germany), while the biomechanical properties of the skin were measured with the aid of an apparatus (Dermaflex, Cortex Technology, Denmark) which records deformations of the skin following application of pressure thereto, measuring its distensibility and elasticity. Skin elasticity and distensibility are correlated with the efficacy of the elastic and collagen fibre network.

The test results are given in table 1, as means of the values measured for each assessment parameter, together with the respective standard deviation and standard error values.

Each mean value was calculated on five measurements taken at the start and at the end of the treatment period (20 days). The units are arbitrary. The prepared product of the invention is indicated in the table as "active cream".

**Table 1**

| | | Mean | Std. deviation | Std. error |
|---|---|---|---|---|
| Elasticity | Placebo | 42.214 | 9.126 | 2.033 |
| | Active cream | 61.571 | 12.143 | 3.040 |
| Hydration | Placebo | 72.613 | 7.342 | 2.301 |
| | Active cream | 85.134 | 5.483 | 1.927 |
| Distensibility | Placebo | 1.430 | 0.213 | 0.042 |
| | Active cream | 1.241 | 0.223 | 0.035 |

Clinical assessment of the patients did not indicate any irritation phenomena and all subjects confirmed the effectiveness and acceptability of the preparation.

The study has therefore shown very significant increases in the hydration and elasticity values of skin (p<0.02) after only 20 days' treatment. The results are of considerable interest because they concur in showing an improved structuring of the fundamental components of the dermis, with increased skin tone. Also to be noted is the substantial increase in skin hydration despite the young age of the tested patients. These results concur with the histological assessment of primary fibroblasts cultures treated with curcumin + zinc-curcumin + FDP.

### TEST 2

### Measurement of oxidative stress

Tests on the capacity of the indicated preparations to prevent oxidative damage, induced in dermal tissue cells, were conducted on human fibroblasts which were isolated then cultivated in a suitable culture medium. Oxidative stress was induced by adding a mixture of 40 mM xanthine and 2 mM hypoxanthine to the culture medium, a mixture with known ability to induce formation of Reactive Oxygen Species (ROS), agents which cause cell damage up to necrosis. The preparations under examination could be added, or not, to the broth at the same time as the xanthine/hypoxanthine mixture. The contact time between the xanthine/hypoxanthine mixture and the preparations was 2 hours, at the end of which the cells were transplanted into a fresh culture medium, i.e. containing neither the stress-inducing mixture nor the substance, then allowed to quiesce for periods of 3 or 24 hours. At the end of the stated period, gene expression of the prostaglandin G/H synthase and cyclooxygenase₂ (COX₂) enzyme was detected which is indicative of the inflammatory state induced in cells by oxidative stress. The content of COX₂ mRNA in cells was then quantified by reverse transcriptase; said content was chiefly increased in cells that had borne oxidative stress the most and lowest in control cells not exposed to oxidative stress. The effectiveness of the different preparations in protecting cultured human fibroblasts from oxidative stress was shown by their ability to maintain COX₂ content as low as and as close to the value found in cells not exposed to stress.

### Synergy between curcumin, zinc and FDP in protection from oxidative stress.

Expression of COX₂ (normalized fluorescence units vs housekeeping gene 18s rRNA) 24 hours after oxidative stress, in "insulted" cells in the presence of curcumin, curcumin complexed with Zn and FDP used separately, and in the presence of the ternary system.

| | COX₂ |
|---|---|
| Non-insulted, untreated cells | 2.12 |
| Insulted, untreated cells | 12.00 |
| | |
| curcumin 3 µM | 5.00 |
| curcumin 6 µM | 4.90 |
| Zn-curcumin complex 3 µM | 4.56 |
| FDP 5 mM | 7.14 |
| curcumin 3 µM + FDP 5 mM | 4.00 |
| curcumin 3 µM + Zn-curcumin complex 3 µM | 4.00 |
| curcumin 3 µM + Zn-curcumin complex 3 µM FDP 5 µM | 3.00 |

### TEST 3

### COX₂ expression after oxidative insult

The previous test was repeated with different compositions and the results measured at 3 hours and 24 hours.

| | 3 hours | 24 hours |
|---|---|---|
| Non-insulted, untreated cells | 2.00 | 2.12 |
| Insulted, untreated cells | 10.00 | 12.00 |
| | | |
| curcumin 3 µM | 5.76 | 5.00 |
| curcumin 6 µM | 5.45 | 4.90 |
| curcumin 9 µM | 5.10 | 4.78 |
| | | |
| Zn-curcumin complex 3 µM | 4.97 | 4.56 |
| Zn-curcumin complex 6 µM | 4.89 | 4.11 |
| Zn-curcumin complex 9 µM | 4.77 | 4.00 |
| | | |
| FDP 5 mM | 7.87 | 7.14 |
| FDP 10 mM | 6.90 | 6.45 |
| | | |
| boswellic acid 100 µM | 7.80 | 8.00 |
| boswellic acid 300 µM | 8.10 | 8.90 |
| curcumin 3 µM + FDP 5 mM | 3.50 | 4.00 |
| curcumin 3 µM + Zn-curcumin complex 3 µM | 3.20 | 2.78 |
| curcumin 9 µM + Zn-curcumin complex 9 µM | 3.30 | 2.90 |
| | | |
| curcumin 3 µM + Zn-curcumin complex 3 µM - FDP 5µm | 3.30 | 3.00 |
| curcumin 9 µM + Zn-curcumin complex 9 µM + FDP 5µm | 3.01 | 2.89 |

From these results the synergistic effect exhibited by FDP and Zn salts on curcumin activity at the cellular level is clear, in the sense that viability of said cells is maintained and reactivated.

## Claims

1. Composition suitable for maintaining skin cells at, or helping to restore skin cells to, their basal physiological state, enabling them to effect a regeneration of the dermis and the epidermis, comprising:
- curcumin,
- fructose 1-6 diphosphate,
- an oxide, or a biologically acceptable salt of a metal able to form, with at least one of said compounds, complexes able to enhance their activity,
in combination with excipients and/or diluents normally used for cosmetic or pharmaceutical-dermatological use.

2. Composition as claimed in claim 1 containing curcumin at concentrations between 0.0005% and 10% on the total weight of the composition.

3. Composition as claimed in claim 1 or claim 2 containing fructose 1-6 diphosphate at concentrations between 0.001% and 25% by weight on the total weight of the composition.

4. Composition as claimed in any one of claims 1-3 comprising oxides or salts of a metal able to form, with at least one of the aforesaid compounds, complexes able to enhance their activity.

5. Composition as claimed in claim 4 wherein said metal is chosen from the group consisting of calcium, magnesium, copper, bismuth, zinc, aluminium, manganese, antimony, tin, gold, silver, chromium, cobalt, vanadium and titanium.

6. Composition as claimed in claim 5 containing metals able to complex curcumin.

7. Composition as claimed in claim 6 wherein the metals are present in quantities such that they completely complex curcumin.

8. Composition as claimed in claim 7 containing complexed curcumin.

9. Composition as claimed in claim 6 wherein the metals are present in quantities such as to partially complex curcumin.

10. Composition as claimed in claim 6 containing a mixture of curcumin and complexed curcumin.

11. Composition as claimed in any one of claims 6-10 wherein the metal is zinc.

12. Composition as claimed in claim 11 wherein the zinc is in the form of zinc acetate or zinc oxide.

13. Composition as claimed in either of claims 11 or 12 wherein complexed curcumin is formed by dissolving the zinc salt in a hydroalcoholic solution and adding curcumin in a 1:2 molar ratio.

14. Composition as claimed in either of claims 12 or 13 wherein complexing occurs by adding curcumin to the composition containing zinc oxide.

15. Composition as claimed in any one of claims 1-14 containing at least one compound chosen from the class consisting of fruit acids (α-hydroxy acids), glucosaminoglycans, urea, urea in protein mixtures, flavones, flavonoids, terpenes, diterpenes, vaseline, saturated and unsaturated fatty acids, lipids, phospholipids, coumarin and derivates, proteins, protides, amino acids, vitamins, in particular D and H group, ceramides, sphingosines, boswellic acids and derivatives, starch and its derivatives, and monosaccharides.

16. Composition as claimed in claim 15 wherein the boswellic acid derivatives are the acetyl and formyl.

17. Composition as claimed in any one of claims 1-16 containing at least one compound chosen from the class consisting of pyrithione and its complex salts, fumaric acid derivatives, Mahonia extracts, anthraquinone derivatives, retinoic acid derivatives, vitamin D derivatives and lactoferrins.

18. Medicament consisting of the composition claimed in any one of claims 1-17 usable in skin diseases where it is essential to block the inflammatory process by modulating calcium and free radical channels caused by oxidative processes, in order to achieve skin regeneration.

19. Medicament consisting of the composition claimed in claim 18 as a coadjuvant for use in the therapeutic treatment of psoriasis.

20. Cosmetic use of the composition claimed in any one of claims 1-17, in particular for the prevention of wrinkle formation and suitable for prevention of elastosis.

## Patentansprüche

1. Zusammensetzung, die geeignet ist, Hautzellen in ihrem basalen physiologischen Zustand zu halten, oder die geeignet ist, dazu beizutragen Hautzellen in ihren basalen physiologischen Zustand zurückzubringen, in dem sie befähigt sind, eine Regeneration der Dermis und der Epidermis zu bewirken, welche umfasst:
- Curcumin,
- Fructose-1,6-diphosphat,
- ein Oxid oder ein biologisch akzeptables Salz eines Metalls, das in der Lage ist, mit wenigstens einer dieser Verbindungen Komplexe zu bilden, die in der Lage sind, deren Aktivität zu verstärken,
in Kombination mit Hilfsstoffen und/oder Verdünnungsmitteln, die normalerweise für eine kosmetische oder pharmazeutisch-dermatologische Anwendungen verwendet werden.

2. Zusammensetzung wie in Anspruch 1 beansprucht, welche Curcumin in Konzentrationen zwischen 0,0005 % und 10 %, bezogen auf das Gesamtgewicht der Zusammensetzung, enthält.

3. Zusammensetzung wie in Anspruch 1 oder Anspruch 2 beansprucht, welche Fructose-1,6-diphosphat in Konzentrationen zwischen 0,001 Gew.-% und 25 Gew.-%, bezogen auf das Gesamtgewicht der Zusammensetzung, enthält.

4. Zusammensetzung wie in einem der Ansprüche 1 bis 3 beansprucht, welche Oxide oder Salze eines Metalls umfasst, das in der Lage ist, mit wenigstens einer der vorgenannten Verbindungen Komplexe zu bilden, die in der Lage sind, deren Aktivität zu verstärken.

5. Zusammensetzung wie in Anspruch 4 beansprucht, worin das Metall ausgewählt ist aus der Gruppe, die besteht aus Calcium, Magnesium, Kupfer, Bismuth, Zink, Aluminium, Mangan, Antimon, Zinn, Gold, Silber, Chrom, Cobalt, Vanadium und Titan.

6. Zusammensetzung wie in Anspruch 5 beansprucht, welche Metalle enthält, die in der Lage sind, mit Curcumin zu komplexieren.

7. Zusammensetzung wie in Anspruch 6 beansprucht, worin die Metalle in Mengen zugegen sind, dass sie das Curcumin vollständig komplexieren.

8. Zusammensetzung wie in Anspruch beansprucht 7, die komplexiertes Curcumin enthält.

9. Zusammensetzung wie in Anspruch 6 beansprucht, worin die Metalle in Mengen zugegen sind, dass sie das Curcumin teilweise komplexieren.

10. Zusammensetzung wie in Anspruch 6 beansprucht, welche eine Mischung aus Curcumin und komplexiertem Curcumin enthält.

11. Zusammensetzung wie in einem der Ansprüche 6 bis 10 beansprucht, worin das Metall Zink ist.

12. Zusammensetzung wie in Anspruch 11 beansprucht, worin das Zink in Form von Zinkacetat oder Zinkoxid vorliegt.

13. Zusammensetzung wie in einem der beiden Ansprüche 11 oder 12 beansprucht, worin das komplexierte Curcumin gebildet wird durch Lösen des Zinksalzes in einer hydroalkoholischen Lösung und Zugeben von Curcumins in einem molaren Verhältnis von 1 : 2.

14. Zusammensetzung wie in einem der beiden Ansprüche 12 oder 13 beansprucht, worin das Komplexieren durch Zugeben von Curcumin zu der Zusammensetzung geschieht, welche Zinkoxid enthält.

15. Zusammensetzung wie in einem der Ansprüche 1 bis 14 beansprucht, die wenigstens eine Verbindung enthält, die ausgewählt ist aus der Klasse, die besteht aus Fruchtsäuren (α-Hydroxysäuren), Glucosaminoglycanen, Harnstoff, Harnstoff in Proteinmischungen, Flavonen, Flavonoiden, Terpenen, Diterpenen, Vaseline, gesättigten und ungesättigten Fettsäuren, Lipiden, Phospholipiden, Coumarin und Derivaten, Proteinen, Peptiden (Protiden), Aminosäuren, Vitaminen, insbesondere der D- und H-Gruppe, Ceramiden, Sphingosinen, Boswelliasäuren und Derivaten, Stärke und ihren Derivaten und Monosacchariden.

16. Zusammensetzung wie in Anspruch 15 beansprucht, worin die Boswelliasäure-Derviate Acetyl- und Formyl-Derivate sind.

17. Zusammensetzung wie in einem der Ansprüche 1 bis 16 beansprucht, die wenigstens eine Verbindung enthält, die ausgewählt ist aus der Klasse, die besteht aus Pyrithion und seine Komplexsalzen, Fumarsäure-Derivaten, Mahonia-Extrakten, Anthrachinon-Derivaten, Retinolsäure-Derivaten, Vitamin D-Derivaten und Lactoferrinen.

18. Medikament, das aus der Zusammensetzung, wie in einem der Ansprüche 1 bis 17 beansprucht, besteht, verwendbar für Hauterkrankungen, bei denen es wesentlich ist, den entzündlichen Prozess zu blockieren durch Modulieren von Kanälen für Calcium und freie Radikale, die durch oxidative Prozesse hervorgerufen werden, um die Hautregeneration zu erreichen.

19. Medikament, das aus der Zusammensetzung, wie in Anspruch 18 beansprucht, besteht als Hilfsstoff zur Verwendung in der therapeutischen Behandlung von Psoriasis.

20. Kosmetische Verwendung der Zusammensetzung, wie in einem der Ansprüche 1 bis 17 beansprucht, insbesondere zur Vorbeugung von Faltenbildung und geeignet zur Vorbeugung von Elastosie.

## Revendications

1. Composition adaptée pour maintenir les cellules cutanées dans, ou pour aider à restaurer les cellules cutanées à, leur état physiologique de base, leur permettant d'effectuer une régénération du derme et de l'épiderme, qui comprend :
- de la curcumine,
- du fructose 1-6 diphosphate,
- un oxyde, ou un sel biologiquement acceptable d'un métal capable de former, avec au moins l'un desdits composés, des complexes capables d'améliorer leur activité,
en combinaison avec des excipients et/ou des diluants normalement utilisés pour une utilisation cosmétique ou pharmacologique-dermatologique.

2. Composition selon la revendication 1, contenant de la curcumine à des concentrations comprises entre 0,0005 % et 10 % du poids total de la composition.

3. Composition selon la revendication 1 ou la revendication 2, contenant du fructose 1-6 diphosphate à des concentrations comprises entre 0,001 % et 25 % en poids du poids total de la composition.

4. Composition selon l'une quelconque des revendications 1 à 3, qui comprend des oxydes ou des sels d'un métal capable de former, avec au moins l'un des composés susmentionnés, des complexes capables d'améliorer leur activité.

5. Composition selon la revendication 4, dans laquelle ledit métal est choisi dans le groupe constitué par le calcium, le magnésium, le cuivre, le bismuth, le zinc, l'aluminium, le manganèse, l'antimoine, l'étain, l'or, l'argent, le chrome, le cobalt, le vanadium et le titane.

6. Composition selon la revendication 5, qui contient des métaux capables de se complexer à la curcumine.

7. Composition selon la revendication 6, dans lequel les métaux sont présents en des quantités telles qu'ils se complexent complètement à la curcumine.

8. Composition selon la revendication 7, qui contient de la curcumine complexée.

9. Composition selon la revendication 6, dans laquelle les métaux sont présents en quantités telles qu'ils se complexent partiellement à la curcumine.

10. Composition selon la revendication 6, contenant un mélange de curcumine et de curcumine complexée.

11. Composition selon l'une quelconque des revendications 6 à 10, dans laquelle le métal est le zinc.

12. Composition selon la revendication 11, dans laquelle le zinc se trouve sous la forme d'acétate de zinc ou d'oxyde de zinc.

13. Composition selon la revendication 11 ou 12, dans laquelle la curcumine complexée est formée par dissolution du sel de zinc dans une solution hydroalcoolique et par addition de curcumine en un rapport molaire de 1/2.

14. Composition selon la revendication 12 ou 13, dans laquelle la complexation a lieu par addition de curcumine à la composition contenant de l'oxyde de zinc.

15. Composition selon l'une quelconque des revendications 1 à 14, contenant au moins un composé choisi dans la classe constituée par les acides de fruit (α-hydroxy acides), les glucosaminoglycanes, l'urée, l'urée dans des mélanges protéiques, les flavones, les flavonoïdes, les terpènes, les diterpènes, la vaseline, les acides gras saturés et insaturés, les lipides, les phospholipides, la coumarine et ses dérivés, les protéines, les protides, les acides aminés, les vitamines, en particulier du groupe D et du group H, les céramides, les sphingosines, les acides boswelliques et leurs dérivés, l'amidon et ses dérivés, et les monosaccharides.

16. Composition selon la revendication 15, dans laquelle les dérivés de l'acide boswellique sont l'acétyle et le formyle.

17. Composition selon l'une quelconque des revendications 1 à 16, qui contient au moins un composé choisi dans la classe constituée par la pyrithione et ses sels complexes, les dérivés de l'acide fumarique, les extraits de Mahonia, les dérivés d'anthraquinone, les dérivés de l'acide rétinoïque, les dérivés de la vitamine D et les lactoferrines.

18. Médicament constitué par la composition selon l'une quelconque des revendications 1 à 17, qui peut être utilisé dans les maladies cutanées dans lesquelles il est essentiel de bloquer le processus inflammatoire par modulation des canaux calciques et des radicaux libres provoqué par des processus oxydatifs, de manière à atteindre une régénération cutanée.

19. Médicament constitué par la composition selon la revendication 18, en tant que co-adjuvant destiné à être utilisé dans le traitement thérapeutique du psoriasis.

20. Utilisation cosmétique de la composition selon l'une quelconque des revendications 1 à 17, en particulier pour la prévention de la formation de rides et adaptée pour la prévention de l'élastose.
